# EUROPÄISCHE PATENTSCHRIFT

(11) **EP 2 147 082 B1**
(45) Veröffentlichungstag und Bekanntmachung des Hinweises auf die Patenterteilung: **02.08.2017**
(21) Anmeldenummer: 08749206.2
(22) Anmeldetag: 29.04.2008
(51) Int. Cl.: C10G 3/00, C10G 50/00, C10L 1/08, C07C 1/20

(54) **VERFAHREN UND ANLAGE ZUR HERSTELLUNG VON SYNTHETISCHEN KRAFTSTOFFEN**
METHOD AND SYSTEM FOR PRODUCING SYNTHETIC FUELS
PROCÉDÉ ET INSTALLATION DE PRODUCTION DE CARBURANTS SYNTHÉTIQUES

(30) Priorität: 11.05.2007 DE 102007022175
(43) Veröffentlichungstag der Anmeldung: 27.01.2010
(73) Patentinhaber: Air Liquide Global E&C Solutions Germany GmbH, 60439 Frankfurt am Main (DE)
(72) Erfinder: ROTHAEMEL, Martin, 60437 Frankfurt am Main (DE); FINCK, Uwe, N-6699 Kjorsvikbugen (NO); DROPSCH, Holger, 61130 Nidderau (DE); BUCHOLD, Henning, 63452 Hanau (DE)
(74) Vertreter: Keil & Schaafhausen Patent- und Rechtsanwälte PartGmbB
(86) Internationale Anmeldenummer: PCT/EP2008/003442
(87) Internationale Veröffentlichungsnummer: WO 2008/138479

(56) Entgegenhaltungen:
- EP-A- 1 410 844
- WO-A-2006/076942
- US-A- 4 899 002
- US-A- 4 929 780
- US-A- 5 177 279
- US-A1- 2002 103 406

## Beschreibung

Die vorliegende Erfindung betrifft ein Verfahren und eine Anlage zur Herstellung von synthetischen Kraftstoffen aus einem Wasserdampf und Oxigenate, wie Methanol und/oder Dimethylether (DME), enthaltenden Eduktgemisch, bei dem in einer ersten Verfahrensstufe das Eduktgemisch an einem Katalysator zu einem Olefine mit vorzugsweise 2 bis 8 Kohlenstoffatomen enthaltenden Kohlenwasserstoffprodukt umgesetzt wird, und in einer zweiten Verfahrensstufe das erhaltene Kohlenwasserstoffprodukt zu höheren Olefinen mit überwiegend mehr als 5, vorzugsweise 10 bis 20 Kohlenstoffatomen, oligomerisiert wird.

Ein derartiges Verfahren zur Herstellung synthetischer Kraftstoffe (so genanntes MtSynfuels-Verfahren; MtSynfuels = Methanol to synthetic fuels) ist aus der WO 2006/076942 A1 bekannt. In einem Olefinreaktor wird hierbei zunächst ein aus Oxigenaten, wie Methanol und/oder DME, und Wasserdampf bestehendes Gemisch an einem Zeolith-Katalysator zu kurzkettigen Olefinen umgesetzt. Als Nebenprodukte entstehen Paraffine und Aromaten. Im Anschluss an die Olefinproduktion wird das im Olefinreaktor erhaltene Olefingemisch verdichtet, teilkondensiert und das Kondensat in einer zweiten Verfahrensstufe zu langkettigen Kohlenwasserstoffen oligomerisiert. Hierbei werden die Aromaten alkyliert. Der oligomerisierte Produktstrom wird in einer anschließenden Trenneinrichtung in einen synthetische Kraftstoffe enthaltenden Produktstrom (LPG, Benzin, Heizgas und nach Hydrierung Diesel/Kerojet) sowie ungesättigte bzw. gesättigte Kohlenwasserstoffe enthaltende Ströme aufgeteilt. Die gesättigten Kohlenwasserstoffe werden zum Olefinreaktor zurückgeführt, während die ungesättigten Kohlenwasserstoffe zum Oligomerisierungsreaktor recycelt werden.

Beim konventionellen MtSynfuels-Verfahren wird die Olefinreaktion üblicherweise bei einem geringen Druck von 1 bis 2 bar betrieben. Dies erfordert große Apparate und Maschinen und führt dadurch zu hohen Investitionskosten.

Die Alkylierung der Aromaten im Oligomerisierungsreaktor führt zu einer Reduzierung der im Diesel erreichbaren Cetanzahl, da die in der nachfolgenden Hydrierung daraus entstehenden Cykloalkane geringe Cetanzahlen aufweisen. Eine zu niedrige Cetanzahl kann zu einem Zündverzug zwischen dem Einspritzen und der Selbstentzündung des Kraftstoffes und damit einer schlagartigen, explosionsartigen Kraftstoffverbrennung mit lautem Verbrennungsgeräusch führen.

Das US Patent 5,177,279 offenbart ein Verfahren zur Herstellung von synthetischen Kraftstoffen aus Oxigenaten und Wasserdampf. In einem Olefinreaktor werden die Oxigenate, insbesondere Methanol, an einem Katalysator zu Olefinen umgesetzt. Das entstehende Kohlenwasserstoffprodukt wird in einen Wasserstrom, einen einem Oligomerisierungsreaktor zugeführten C₂-C₅-Olefinenstrom und einen abgezogenen C₃-C₉-Kohlenwasserstoffstrom aufgetrennt. Letzterer Strom wird in einer weiteren Trenneinrichtung in einen C₆-Strom und einen C₇₊-Strom aufgetrennt. Der C₆₋-Strom wird zusammen mit dem aus der ersten Trenneinrichtung abgezogenen C₂-C₅-Olefinstrom dem Oligomerisierungsreaktor zugeführt. Nach der Oligomerisierung wird der Produktstrom weiter aufgetrennt, wobei ein C₂₋-Strom zu dem Oligomerisierungsreaktor zurückgeführt wird. Eine Weiterverarbeitung der erhaltenen Produktströme zu Diesel ist nicht vorgesehen. Vielmehr ist das Verfahren auf die Herstellung eines Benzinproduktes gerichtet.

Die US-Patente 4,929,780 und 4,899,002 offenbaren ähnliche Verfahren.

Aufgabe der Erfindung ist es, ein Dieselprodukt mit besserer Qualität insbesondere eine höhere Cetanzahl herzustellen.

Diese Aufgabe wird mit der Erfindung durch ein Verfahren mit den Merkmalen des Anspruchs 1 und eine Anlage mit den Merkmalen des Anspruchs 8.

Gemäß der Erfindung wird die Olefinerzeugung in der ersten Verfahrensstufe bei einem Druck von mehr als 2 bar, vorzugsweise 2 bis 10 bar, insbesondere 3 bis 8 bar, durchgeführt. Durch eine Erhöhung des Druckes der Olefinreaktion kann der Querschnitt des Olefinreaktors sowie der angeschlossenen Apparate und Armaturen (Wärmeübertrager, Ventile, Rohrleitungen) deutlich verringert werden. Dies führt zu einer entsprechenden Verringerung der Investitionskosten.

In Weiterbildung der Erfindung wird aus der gasförmigen Phase des Kohlenwasserstoffproduktes vor Einleitung in die zweite Verfahrensstufe die Benzinfraktion abgetrennt und dem Benzinproduktstrom zugeführt. Dies verbessert die Qualität des Benzinprodukts.

Gemäß einer bevorzugten Ausführungsform der Erfindung erfolgt die Trennung der flüssigen Phase des Kohlenwasserstoffproduktes nach der ersten Verfahrensstufe destillativ, wobei erfindungsgemäß das Kopfprodukt der Destillation, bei dem es sich um das an C₆₋-Kohlenwasserstoffen reiche Gemisch handelt, der zweiten Verfahrensstufe zugeführt wird.

Am Sumpfprodukt der Destillation erhält man das C₇₊-Kohlenwasserstoffe und Aromaten enthaltende Gemisch, welches in Weiterbildung der Erfindung einem nach dem Oligomerisierungsreaktor abgetrennten Benzinproduktstrom zugegeben wird.

Wird aus dem in der zweiten Verfahrensstufe erhaltenen Kohlenwasserstoffprodukt ein Dieselproduktstrom abgetrennt, so wird in Weiterbildung der Erfindung das Sumpfprodukt der Destillation nach Hydrierung dem Dieselproduktstrom zugegeben.

Wird aus dem in der zweiten Verfahrensstufe erhaltenen Kohlenwasserstoffprodukt ein Benzinproduktstrom abgetrennt, so wird des aus dem Kopfdestillat und/oder wenigstens einem Seitenabzug der Destillation erhaltene Produkt erfindungsgemäß dem Benzinproduktstrom zugeführt.

Die Erfindung betrifft auch eine Anlage zur Herstellung von synthetischen Kraftstoffen mit den Merkmalen des Anspruchs 8, die insbesondere zur Durchführung des erfindungsgemäßen Verfahrens geeignet ist.

Erfindungsgemäß ist die zweite Trenneinrichtung eine Destillationskolonne, deren Kopfbereich mit dem Eintritt des Oligomerisierungsreaktors verbunden ist. Weiterbildungen, Vorteile und Anwendungsmöglichkeiten der Erfindung ergeben sich auch aus der nachfolgenden Beschreibung von Ausführungsbeispielen und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.
- Fig. 1: zeigt schematisch eine zur Durchführung des erfindungsgemäßen Verfahrens geeignete Anlage,
- Fig. 2.1: zeigt die Abhängigkeit der gemessenen Cetanzahlen (IP 498) des Dieselproduktes nach Hydrierung von dem Aromaten-Massenstrom im Feed des Oligomerisierungsreaktors und
- Fig. 2.2: zeigt die zeitliche Abnahme des Aromatengehaltes im Recycle des Oligomerisierungsreaktors.

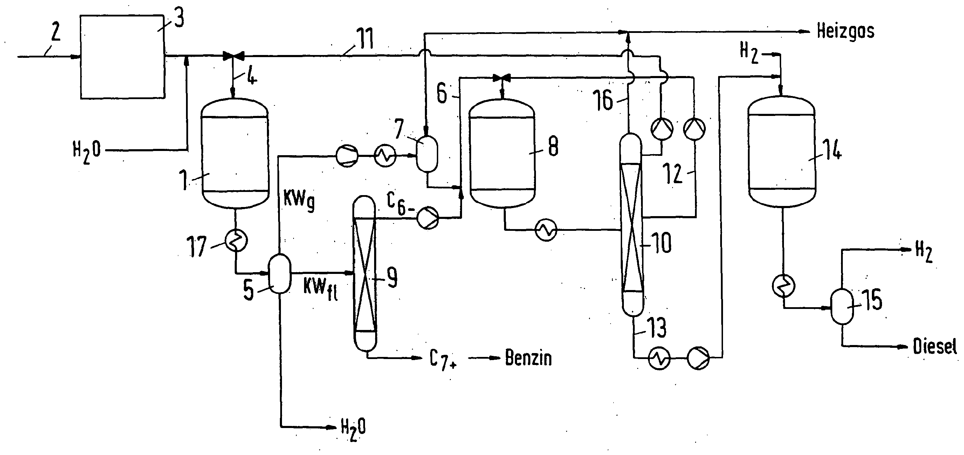

Die in Fig. 1 dargestellte Anlage umfasst zunächst einen Olefinreaktor 1, der einen Katalysator auf Basis von formselektivem Zeolith, vorzugsweise ein Aluminiumsilikat-Zeolith vom Pentasiltyp und besonders bevorzugt ZSM-5, oder Katalysatoren basierend auf Silikalit oder Aluminiumphosphat (SAPO) enthält. Der Olefinreaktor 1 ist vorzugsweise ein mehrstufiger adiabater Festbettreaktor, doch ist es auch möglich, lediglich einen einstufigen Reaktor oder mehrere parallele oder in Reihe angeordnete Reaktoren zu verwenden.

Bei Betrieb der Anlage wird über eine Methanolzufuhrleitung 2 herangeführtes Methanol in einem nicht dargestellten Wärmetauscher auf eine Temperatur von vorzugsweise 200 bis 350°C erhitzt und dadurch verdampft, bevor der Methanoldampf in einem DME-Reaktor 3 an einem geeigneten Dehydrierungskatalysator, beispielsweise Aluminiumoxid, zumindest teilweise zu Dimethylether und Wasser umgesetzt wird. Das aus dem DME-Reaktor 3 abgezogene Methanol/ Dimethylethergemisch wird über eine Leitung 4 dem Olefinreaktor 1 zugeführt. Hierbei kann zunächst ein Teilstrom des Eduktgemisches über eine Abzweigleitung (nicht dargestellt) abgezweigt und nach Abkühlung in einzelnen Teilströmen gasförmig auf die einzelnen Reaktorstufen des mehrstufigen Olefinreaktors 1 aufgegeben werden. Der Hauptstrom wird auf die erste Stufe des Olefinreaktors 1 aufgegeben. Vorzugsweise beträgt die Eintrittstemperatur in die erste Stufe des Olefinreaktors 1 zwischen 350 und 500°C. Das Gewichtsverhältnis Wasser zu Methanoläquivalent in dem Eduktgemisch liegt bevorzugt zwischen 0,25 : 1 und 10 : 1. Ein "Methanoläquivalent" entspricht hierbei nach der Gleichung 2 CH₃OH → CH₃-O-CH₃ + H₂O einem halben Mol Dimethylether. Es ist möglich, anstelle des Wasserdampf-/Methanol-/Dimethylethergemisches in dem Reaktor 1 ausschließlich Methanol oder Dimethylether in Kombination mit Wasserdampf als Edukt einzusetzen.

Der Olefinreaktor 1 wird mit einem erhöhten Druck > 2 bar, vorzugsweise 2 bis 10 bar, insbesondere 3 bis 8 bar, betrieben. Im Katalysatorbereich des Olefinreaktors 1 liegen die Temperaturen zwischen 300 und 600°C.

Das in dem Olefinreaktor 1 gebildete Reaktionsgemisch besteht bei einem Betrieb des Olefinreaktors bei niedrigerem Druck vornehmlich aus C₂-C₄-Olefinen, C₅₊-Benzinkohlenwasserstoffen und Wasserdampf. Durch die Erhöhung des Druckes in dem Olefinreaktor 1 auf > 2 bar verschiebt sich das Produktspektrum zu langkettigen Olefinen und Paraffinen (bis etwa C₈) sowie in geringerem Umfang zu Aromaten.

Im Anschluss an den Olefinreaktor 1 wird das erhaltene Reaktionsgemisch nach Abkühlung über einen Wärmetauscher 17 in einer ersten Trenneinrichtung 5 (3-Phasenabscheider) in eine wässrige Phase, einen gasförmigen Kohlenwasserstoffstrom KW_{g} und einen flüssigen Kohlenwasserstoffstrom KW_{fl} aufgetrennt. Ein Teil der wässrigen Phase kann hierbei zum Eingang des Olefinreaktors 1 rezirkuliert werden.

Die gasförmigen Kohlenwasserstoffe werden nach Verdichtung und Teilkondensation über eine Leitung 6, ggf. nach Abtrennung einer Fraktion gasförmiger Kohlenwasserstoffe in einer Trenneinrichtung 7, dem Eintritt eines Oligomerisierungsreaktors 8 zugeführt.

Die in der ersten Trenneinrichtung 5 erhaltenen flüssigen Kohlenwasserstoffe werden einer zweiten Trenneinrichtung 9 in Form einer Destillationskolonne zugeführt, in welcher sie in einen C₆₋-Kohlenwasserstoffstrom und ein C₇₊-Kohlenwasserstoff und Aromaten enthaltendes Gemisch aufgetrennt werden. Am Kopf der Destillationskolonne 9 wird der C₆₋-Kohlenwasserstoffstrom abgezogen und dem Eintritt des mehrstufigen Oligomerisierungsreaktors 8 zugeführt. Das Gemisch aus C₇₊-Kohlenwasserstoffen und Aromaten wird abgezogen und kann einem Benzinproduktstrom zugeführt werden.

Das der ersten Stufe des mehrstufigen Oligomerisierungsreaktors 8 zugeführte Kohlenwasserstoffgemisch wird in diesem bei Temperaturen zwischen 200 und 450°C und bei einem Druck von 40 bis 100 bar in Gegenwart von Zeolith-Katalysatoren vom Pentasiltyp oligomerisiert. Die durch die Oligomerisierung entstehenden Olefine weisen überwiegend mehr als 5, vorzugsweise zwischen 10 und 20, Kohlenstoffatome auf. Das erhaltene Gemisch wird in einer anschließenden Destillationskolonne 10 (dritte Trenneinrichtung) aufgearbeitet, wobei ein Destillat abgetrennt wird, welches nach Hydrierung das Dieselprodukt bildet. Weiter werden zwei Kohlenwasserstoffströme unterschiedlicher Zusammensetzung bereitgestellt. Ein paraffinreicher Kohlenwasserstoffstrom wird über eine Leitung 11 zum Olefinreaktor 1 rezirkuliert, während ein olefinreicher Strom über eine Leitung 12 wieder dem Eintrittsbereich des Oligomerisierungsreaktors 8 zugeführt wird. Dem olefin- und paraffinreichen Kohlenwasserstoffstrom können Teilmengen zur Bereitstellung des Benzinprodukts entnommen werden.

Das über die Leitung 13 aus dem Sumpf der Destillationskolonne 10 abgezogene Flüssigprodukt wird nach Zumischung von Wasserstoff einer Hydrieranlage 14 zugeführt, in der die ungesättigten Kohlenwasserstoffe in Diesel umgewandelt werden. In einer anschließenden Trenneinrichtung 15 werden die Dieselprodukte von überschüssigem Wasserstoff getrennt. Der größte Teil des überschüssigen Wasserstoffs kann zum Hydrierreaktor 14 recycelt werden.

Am Kopf der dritten Trenneinrichtung 10 wird über eine Leitung 16 ein Heizgasstrom abgezogen, wobei je nach Gestaltung der dritten Trenneinrichtung 10 auch weitere Produktströme, wie LPG oder Benzin abgezogen werden können.

Durch die Abtrennung der C₇₊-Komponenten in der zweiten Trenneinrichtung wird an sich die Ausbeute an Dieselkraftstoff verringert. Dieser Effekt wird jedoch durch die Erhöhung der Benzinausbeute und insbesondere die Steigerung der Qualität sowohl des Benzin- als auch des Dieselproduktes, für welche sich höhere Oktan- bzw. Cetanzahlen ergeben, überkompensiert. Die Ausschleusung der Aromaten in der zweiten Trenneinrichtung erhöht die Cetanzahl im Dieselprodukt. Da die ausgeschleusten Aromaten stattdessen dem Benzinprodukt zugegeben werden, wird gleichzeitig dessen Oktanzahl erhöht.

### Beispiele:

Beispiel 1: Erhöhung der Cetanzahlen des Dieselproduktes durch Abtrennung der C₇₊-Kohlenwasserstoffe aus dem Feed des Oligomerisierungsreaktors

Aromatische Kohlenwasserstoffe weisen im Vergleich zu nichtaromatischen Kohlenwasserstoffen gleicher Kohlenstoffzahl eine erheblich kleinere Cetanzahl auf. Zahlenbeispiele zur Veranschaulichung dieses Effektes sind nachfolgend in Tabelle 1.1 zusammengestellt.

**Tabelle 1.1: Cetanzahlen (CN) für aromatische und nichtaromatische Kohlenwasserstoffe (KW) gleicher Kohlenstoffzahl.**

| **C-Zahl** | **Aromatischer KW / CN** | | **Nichtaromatischer KW / CN** | |
|---|---|---|---|---|
| 6 | Benzol | 0 | n-Hexan | 42...45 |
| 12 | n-Hexylbenzol | 26 | n-Dodecan | 80...88 |
| 16 | n-Octylxylol | 20 | n-Hexadecan | 100 |
| | | | (Cetan, per Definition) | |

(Quelle: Murphy, M. J. et al., Compendium of Experimental Cetane Number Data, National Renewable Energy Laboratory (www.nrel.gov), NREL/SR-540-36805, September 2004)

Eine möglichst vollständige Entfernung aromatischer Kohlenwasserstoffe sowohl aus Frischfeed als auch aus dem zum Oligomerisierungsreaktor zurückgeführten KW-Recycle ist daher von großer Bedeutung zur Erhöhung der Cetanzahl des Dieselproduktes.

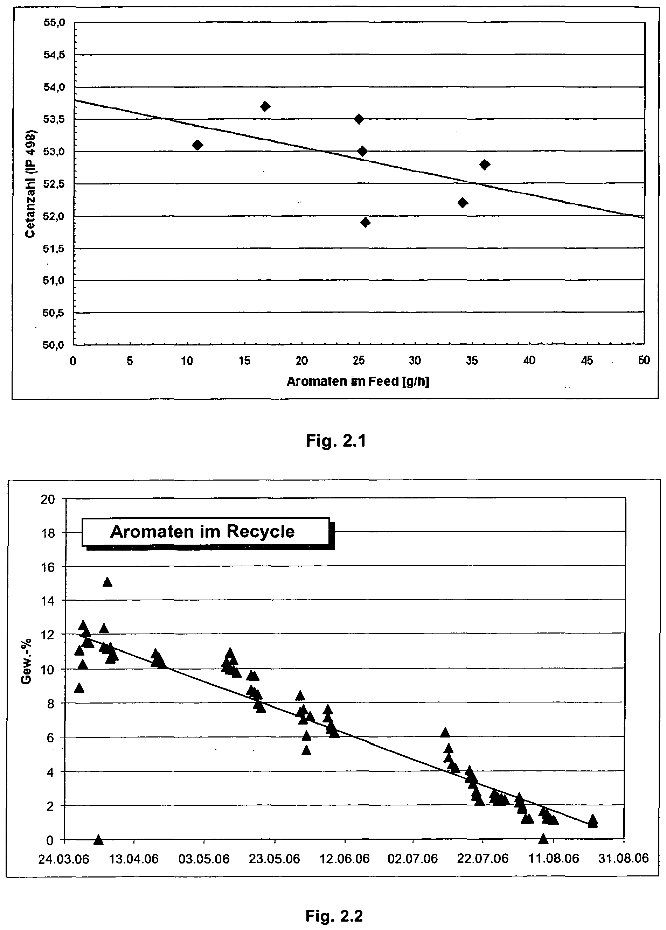

In Fig. 2.1 ist der Zusammenhang zwischen Aromaten-Massenstrom im Feed des Oligomerisierungsreaktors und den gemessenen Cetanzahlen des Dieselproduktes nach Hydrierung graphisch dargestellt. Durch destillative Abtrennung und Ausschleusung des C₇₊-Schnittes aus dem Feed des Oligomerisierungsreaktors konnte eine sofortige Abnahme des Aromaten-Massenstroms im Frischfeed und eine kontinuierliche Abnahme im Recycle des Oligomerisierungsreaktors bis auf Spuren erreicht werden, Fig. 2.2.

### Beispiel 2: Oktanzahlen der Benzinproduktströme

Vor Inbetriebnahme der Aromatenkolonne bildeten die über Leitung 11 am Kolonnenkopf und über Leitung 12 an einem Seitenabzug der Dieselkolonne 10 entnommenen, flüssigen Kohlenwasserstoffströme das Benzinprodukt. Nach Inbetriebnahme der destillativen Trennung des flüssigen Kohlenwasserstoffproduktes aus dem Olefinreaktor 1 steht für das Benzinprodukt ein weiterer, aromatenreicher Teilstrom mit hoher Oktanzahl zur Verfügung. Typische Oktanzahlen der drei Teilströme sind in der nachfolgenden Tabelle 2.1 zusammengestellt.

**Tabelle 2.1: Wichtige Eigenschaften der Teilströme des Benzinproduktes**

| **Teilstrom** | **Oktanzahl (RON)** |
|---|---|
| Kopfdestillat (11) | 81,4 |
| Seitenabzug (12) | 83,0 |
| Sumpf Aromatenkolonne (C₇₊) | 96,4 |

Gemäß der für Ottokraftstoffe geltenden DIN EN 228 ist der Aromatengehalt auf 42 Vol.-% begrenzt, wobei für den Benzolgehalt (C₆H₆) ein Grenzwert von 1 Vol.-% gilt. Wie Tabelle 2.2 zeigt, ist der Sumpf der Aromatenkolonne (C₇₊) praktisch benzolfrei. Er kann daher dem Benzinprodukt bis zur Erreichung des Grenzwertes für den Gesamt-Aromatengehalt zugegeben werden.

**Tabelle 2.2: Typische Verteilung aromatischer Kohlenwasserstoffe im Sumpf der Aromatenkolonne (C₇₊)**

| | | | | | | | | |
|---|---|---|---|---|---|---|---|---|
| **C-Zahl** | **6** | **7** | **8** | **9** | **10** | **11** | **12** | **Total** |
| **Gew.-%** | **0,0** | **4,7** | **34,9** | **27,9** | **7,6** | **0,5** | **0,0** | **75,5** |

### Beispiel 3: Mengenänderung des Diesel- und des Benzinproduktes durch Abtrennung der C₇₊-Kohlenwasserstoffe aus dem Feed des Oligomerisierungsreaktors

Durch destillative Abtrennung und Ausschleusung des C₇₊-Schnittes aus dem flüssigen Kohlenwasserstoffprodukt des Olefinreaktors 1 wird der dem Oligomerisierungsreaktor 8 zugeführte Gesamtfeed mengenmäßig reduziert. Da dieser Schnitt dem Benzinprodukt zugegeben wird, vergrößert sich seine Menge entsprechend. Typische Produktmengen ohne bzw. mit Fraktionierung des Olefinreaktor-Flüssigproduktes werden nachfolgend in Tabelle 3.1 verglichen.

Bezogen auf 1000 g/h Frischfeed werden ohne Fraktionierung 622 g/h Dieselvorprodukt erhalten, im Fall mit Fraktionierung 616 g/h. Folglich ist der Einfluss der Fraktionierung auf die Dieselausbeute vergleichsweise gering. Im Gegenzug steigt die Benzinmenge von 188 g/h (ohne Fraktionierung) auf 292 g/h (mit Fraktionierung) pro 1000 g/h Frischfeed.

**Tabelle 3.1: Typische Feed- und Produktmengen des Oligomerisierungsreaktors ohne bzw. mit Fraktionierung des flüssigen Kohlenwasserstoffproduktes aus dem Olefinreaktor**

| **Strom Bezeichung** | **Ohne Fraktionierung Massenstrom / g/h** | **Mit Fraktionierung Massenstrom / g/h** |
|---|---|---|
| ***Feedströme*** | | |
| Frischfeed ^{#)} | 308 | 229 |
| Recycle | 903 | 926 |

| ***Produktströme*** | | |
|---|---|---|
| Sumpf Aromatenkolonne (C₇₊) | 0 | 32 |
| Kopfgas (16) | 43 | 46 |
| Kopfdestillat (11) + Seitenabzug (12) | 961 | 961 |
| Benzinpurge | 58 | 35 |
| Sumpf Dieselkolonne (13) | 192 | 141 |

| | | |
|---|---|---|
| #) Frischfeed zum Oligomerisierungsreaktor = gasförmiges Produkt des Olefinreaktors + Kopfprodukt Aromatenkolonne | | |

### Bezugszeichenliste:

- 1: Olefinreaktor
- 2: Methanolzufuhrleitung
- 3: DME-Reaktor
- 4: Leitung
- 5: erste Trenneinrichtung
- 6: Leitung
- 7: Trenneinrichtung
- 8: Oligomerisierungsreaktor
- 9: zweite Trenneinrichtung
- 10: dritte Trenneinrichtung
- 11: Leitung
- 12: Leitung
- 13: Leitung
- 14: Hydrieranlage
- 15: Trenneinrichtung
- 16: Leitung
- 17: Wärmetauscher

- KW_{g}: gasförmiger Kohlenwasserstoffstrom
- KW_{fl}: flüssiger Kohlenwasserstoffstrom

## Patentansprüche

1. Verfahren zur Herstellung von synthetischen Kraftstoffen aus einem Wasserdampf und Oxigenate, wie Methanol und/oder Dimethylether, enthaltenden Eduktgemisch, bei dem
in einer ersten Verfahrensstufe das Eduktgemisch bei einem Druck von mehr als 2 bar und einer Temperatur zwischen 300 und 600°C an einem Katalysator zu einem Olefine mit vorzugsweise 2 bis 8 Kohlenstoffatomen enthaltenden Kohlenwasserstoffprodukt umgesetzt wird, und
in einer zweiten Verfahrensstufe das erhaltene Kohlenwasserstoffprodukt bei einem Druck von 40 bis 100 bar und einer Temperatur zwischen 200 und 450°C in Gegenwart von Zeolith-Katalysatoren vom Pentasiltyp zu höheren Olefinen mit überwiegend mehr als 5, vorzugsweise 10 bis 20 Kohlenstoffatomen, oligomerisiert wird,
wobei das in der ersten Verfahrensstufe erhaltene Kohlenwasserstoffprodukt vor Einleitung in die zweite Verfahrensstufe in eine flüssige und eine gasförmige Phase aufgetrennt wird,
wobei die gasförmige Phase des Kohlenwasserstoffprodukts der zweiten Verfahrensstufe zugeführt wird,
wobei die flüssige Phase des Kohlenwasserstoffprodukts in einer zweiten Trenneinrichtung in ein an C₆₋-Kohlenwasserstoffen reiches Gemisch und ein C₇₊-Kohlenwasserstoffe und Aromaten enthaltendes Gemisch aufgetrennt wird, wobei das an C₆₋-Kohlenwasserstoffen reiche Gemisch der zweiten Verfahrensstufe zugeführt wird,
wobei das in der zweiten Verfahrensstufe erhaltene Gemisch in einer dritten Trennrichtung in Form einer Destillationskolonne in ein Destillat, einen paraffinreichen Kohlenwasserstoffstrom und einen olefinreichen Kohlenwasserstoffstrom aufgetrennt wird,
wobei das Destillat nach Zugabe von Wasserstoff in einer Hydrieranlage zu Diesel umgewandelt wird,
wobei der paraffinreiche Kohlenwasserstoffstrom zu der ersten Verfahrensstufe zurückgeführt wird, und
wobei der olefinreiche Kohlenwasserstoffstrom zum Eintrittsbereich der zweiten Verfahrensstufe zurückgeführt wird.

2. Verfahren nach Anspruch 1, **dadurch gekennzeichnet, dass** die Olefinerzeugung in der ersten Verfahrensstufe bei einem Druck von mehr als 2 bar, vorzugsweise 2 bis 10 bar, insbesondere 3 bis 8 bar, durchgeführt wird.

3. Verfahren nach Anspruch 1 oder 2, **dadurch gekennzeichnet, dass** das C₇₊-Kohlenwasserstoffe und Aromaten enthaltende Gemisch mindestens teilweise dem Benzinprodukt zugeführt wird.

4. Verfahren nach einem der vorhergehenden Ansprüche, **dadurch gekennzeichnet, dass** die Trennung der flüssigen Phase des Kohlenwasserstoffprodukts nach der ersten Verfahrensstufe destillativ erfolgt.

5. Verfahren nach Anspruch 4, **dadurch gekennzeichnet, dass** das Kopfprodukt der Destillation der zweiten Verfahrensstufe zugeführt wird.

6. Verfahren nach Anspruch 4 oder 5, wobei aus dem in der zweiten Verfahrensstufe erhaltenen Kohlenwasserstoffprodukt ein Dieselproduktstrom abgetrennt wird, **dadurch gekennzeichnet, dass** das Sumpfprodukt der Destillation nach Hydrierung wenigstens teilweise dem Dieselproduktstrom zugegeben wird.

7. Verfahren nach einem der Ansprüche 4 bis 6, wobei aus dem in der zweiten Verfahrensstufe erhaltenen Kohlenwasserstoffprodukt ein Benzinproduktstrom abgetrennt wird, **dadurch gekennzeichnet, dass** das aus dem Kopfdestillat und/oder einem Seitenabzug der Destillation erhaltene Produkt wenigstens teilweise dem Benzinproduktstrom zugegeben wird.

8. Anlage zur Herstellung von synthetischen Kraftstoffen aus einem Wasserdampf und Oxigenate, wie Methanol und/oder Dimethylether, enthaltenden Eduktgemisch, insbesondere geeignet zur Durchführung eines Verfahrens nach einem der vorhergehenden Ansprüche,
mit wenigstens einem katalytischen Olefinreaktor (1) zur Umsetzung des Eduktgemisches zu einem Olefine mit vorzugsweise 2 bis 8 Kohlenstoffatomen enthaltenden Kohlenwasserstoffprodukt,
mit wenigstens einem dem Olefinreaktor (1) nachgeschalteten Oligomerisierungsreaktor (8) zur Umsetzung des erhaltenen Kohlenwasserstoffprodukts zu langkettigen Kohlenwasserstoffen,
mit einer ersten Trenneinrichtung (5) zur Auftrennung des in dem Olefinreaktor (1) erhaltenen Kohlenwasserstoffprodukts in eine gasförmige und eine flüssige Phase, wobei die gasförmige Phase über eine Leitung mit dem Eintritt des Oligomerisierungsreaktor (8) verbunden ist,
mit einer zweiten Trenneinrichtung (9) zur Auftrennung der flüssigen Phase des Kohlenwasserstoffprodukts in ein an C₆₋-Kohlenwasserstoffen reiches Gemisch und ein C₇₊-Kohlenwasserstoffe und Aromaten enthaltendes Gemisch, wobei der Kopfbereich der zweiten Trenneinrichtung (9) über eine Leitung (6) mit dem Eintritt des Oligomerisierungsreaktors (8) verbunden ist, und
mit einer dritten Trenneinreichung (10) in Form einer Destillationskolonne (10)zur Auftrennung des im Oligomerisierungsreaktor (8) erhaltenen Gemischs in ein Destillat, einen paraffinreichen Kohlenwasserstoffstrom und einen olefinreichen Kohlenwasserstoffstrom,
wobei der Sumpf der dritten Trenneinrichtung (10) über eine Leitung (13) mit einem Hydrierreaktor (14) verbunden ist, in welchem aus dem Destillat unter Zugabe von Wasserstoff Diesel hergestellt wird,
wobei der Kopfbereich der dritten Trenneinrichtung (10) über eine Leitung (11) mit dem Eintritt des Olefinreaktors (1) verbunden ist, und
wobei die dritte Trenneinrichtung (10) über eine Leitung (12) mit dem Eintritt des Oligomerisierungsreaktors (8) verbunden ist.

9. Anlage nach Anspruch 8, **dadurch gekennzeichnet, dass** die zweite Trenneinrichtung (9) eine Destillationskolonne ist.

## Claims

1. Method for producing synthetic fuels from a reactant mixture containing steam and oxygenates such as methanol and/or dimethyl ether, in which
in a first method stage the reactant mixture is converted into a hydrocarbon product containing olefins having preferably 2 to 8 carbon atoms at a pressure greater than 2 bar and a temperature between 300 and 600 °C on a catalyst, and
in a second method stage, the hydrocarbon product obtained is oligomerised to yield higher olefins predominantly with more than 5 carbon atoms, preferably 10 to 20 carbon atoms, at a pressure from 40 to 100 bar and a temperature between 200 and 450 °C in the presence of zeolite catalysts of the pentasil type,
wherein the hydrocarbon product obtained in the first method stage is separated into a liquid phase and a gas phase before it is introduced into the second method stage,
wherein the gas phase of the hydrocarbon product is introduced into the second method stage,
wherein the liquid phase of the hydrocarbon product is separated into a mixture rich in C₆ hydrocarbons and a mixture containing C₇₊ hydrocarbons and aromatics in a second separating device,
wherein the mixture rich in C₆ hydrocarbons is fed to the second method stage,
wherein the mixture obtained in the second method stage is separated into a distillate, a paraffin-rich hydrocarbon stream and an olefin-rich hydrocarbon stream in a third separating device in the form of a distillation column,
wherein the distillate is converted into diesel after the addition of hydrogen in a hydrogenation plant,
wherein the paraffin-rich hydrocarbon stream is returned to the first method stage, and
wherein the olefin-rich hydrocarbon stream is returned to the inlet region of the second method stage.

2. Method according to claim 1, **characterised in that** the olefin production is carried out in the first method stage at a pressure of more than 2 bar, preferably 2 to 10 bar, particularly 3 to 6 bar.

3. Method according to claim 1 or 2, **characterised in that** at least a part of the mixture containing C₇₊ hydrocarbons and aromatics is fed to a fuel product.

4. Method according to any one of the preceding claims, **characterised in that** the separation of the liquid phase of the hydrocarbon product after the first method stage is carried out by distillation.

5. Method according to claim 4, **characterised in that** the overhead product of the distillation is fed to the second method stage.

6. Method according to claim 4 or 5, wherein a diesel product stream is separated from the hydrocarbon product obtained in the second method stage, **characterised in that** at least a part of the bottom product from the distillation is added to the diesel product stream after hydrogenation.

7. Method according to any one of claims 4 to 6, wherein a fuel product stream is separated from the hydrocarbon product obtained in the second method stage, **characterised in that** at least a part of the product obtained from the overhead distillate and/or a sidestream of the distillation is added to the fuel product stream.

8. System for producing synthetic fuels from a reactant mixture containing steam and oxygenates such as methanol and/or dimethyl ether, particularly designed to carry out a method according to any one of the preceding claims,
having at least one catalytic olefin reactor (1) for converting the reactant mixture into a hydrocarbon product containing olefins having preferably 2 to 8 carbon atoms,
having at least one oligomerisation reactor (8) connected downstream of the olefin reactor (1) for converting the hydrocarbon product obtained into long-chain hydrocarbons,
having a first separating device (5) for separating the hydrocarbon product obtained in the olefin reactor (1) into a gas phase and a liquid phase, wherein the gas phase is connected to the inlet of the oligomerisation reactor (8) via a supply line,
having a second separating device (9) for separating the liquid phase of the hydrocarbon product into a mixture rich in C₆ hydrocarbons and a mixture containing C₇₊ hydrocarbons and aromatics, wherein the overhead region of the second separating device (9) is connected to the inlet of the oligomerisation reactor (8) via a supply line (6), and
having a third separating device (10) in the form of a distillation column (10) for separating the mixture otained in the oligomerisation reactor (8) into a distillate, a paraffin-rich hydrocarbon stream and an olefin-rich hydrocarbon stream,
wherein the bottom region of the third separating device (10) is connected via a supply line (13) to a hydrogenation reactor (14), in which diesel is produced from the distillate with the addition of hydrogen,
wherein the overhead region of the third separating device (10) is connected to the inlet of the olefin reactor (1) via a supply line (11),
wherein the third separating device (10) is connected to the inlet of the oligomerisation reactor (8) via a supply line (12).

9. Device according to claim 8, **characterised in that** the second separating device (9) is a distillation column.

## Revendications

1. Procédé destiné à produire des carburants synthétiques à partir d'un mélange d'éduits contenant de la vapeur d'eau et des oygénates, comme le méthanol et/ou l'éther diméthylique, lors duquel
dans une première étape de procédé, sur un catalyseur, on transforme le mélange d'éduits à une pression de plus de 2 bar et à une température comprise entre 300 et 600°C en un produit hydrocarboné contenant des oléfines avec de préférence de 2 à 8 atomes de carbone, et
dans une deuxième étape de procédé, on oligomérise le produit hydrocarboné obtenu à une pression de 40 à 100 bar et à une température comprise entre 200 et 450°C en présence de catalyseurs zéolithiques du type pentasile en des oléfines supérieures, avec majoritairement plus de 5, de préférence de 10 à 20 atomes de carbone,
avant l'introduction dans la deuxième étape de procédé, le produit hydrocarboné obtenu dans la première étape de procédé étant séparé en une phase liquide et une phase gazeuse,
la phase gazeuse du produit hydrocarboné de la deuxième étape de procédé étant alimentée,
dans un deuxième dispositif de séparation, la phase liquide du produit hydrocarboné étant séparée en un mélange riche en hydrocarbures en C₆ et en un mélange contenant des hydrocarbures en C₇₊ et des aromates,
le mélange riche en hydrocarbures en C₆ étant alimenté vers la deuxième étape de procédé,
dans un troisième dispositif de séparation sous la forme d'une colonne de distillation, le mélange obtenu dans la deuxième étape de procédé étant séparé en un distillat, en un flux d'hydrocarbure riche en paraffine et en un flux d'hydrocarbure riche en oléfines,
après ajout d'hydrogène, dans une installation d'hydratation, le distillat étant transformé en diesel,
le flux d'hydrocarbure riche en paraffine étant renvoyé dans la première étape de procédé et
le flux d'hydrocarbure riche en oléfines étant renvoyé dans la zone d'entrée de la deuxième étape de procédé.

2. Produit selon la revendication 1, **caractérisé en ce que** la génération d'oléfines est réalisée dans la première étape de procédé à une pression de plus de 2 bar, de préférence de 2 à 10 bar, notamment de 3 à 8 bar.

3. Produit selon la revendication 1 ou 2, **caractérisé en ce que** le mélange contenant des hydrocarbures en C₇₊ et des aromates est alimenté au moins en partie dans le produit d'essence.

4. Produit selon l'une quelconque des revendications précédentes, **caractérisé en ce que** la séparation de la phase liquide du produit hydrocarboné s'effectue par distillation, après la première étape de procédé.

5. Produit selon la revendication 4, **caractérisé en ce que** le produit de la distillation est alimenté vers la deuxième étape de procédé.

6. Produit selon la revendication 4 ou 5, à partir du produit hydrocarboné obtenu dans la deuxième étape de procédé étant séparé un flux de produit diesel, **caractérisé en ce qu'**après l'hydratation, le produit de pied de colonne de la distillation est ajouté au moins en partie au flux de produit diesel.

7. Procédé selon l'une quelconque des revendications 4 à 6, à partir du produit hydrocarboné obtenu dans la deuxième étape de procédé étant séparé un flux de produit d'essence, **caractérisé en ce que** le produit obtenu à partir du distillat de tête et/ou d'un soutirage latéral de la distillation est ajouté au moins en partie au flux de produit d'essence.

8. Installation destinée à produire des carburants synthétiques à partir d'un mélange d'éduits contenant de la vapeur d'eau et des oygénates, comme le méthanol et/ou l'éther diméthylique, notamment adapté pour réaliser un procédé selon l'une quelconque des revendications précédentes,
avec au moins un réacteur catalytique pour oléfines (1) pour transformer le mélange d'éduits en un produit hydrocarboné contenant une oléfine avec de préférence de 2 à 8 atomes de carbone,
avec au moins un réacteur d'oligomérisation (8) monté en aval du réacteur pour oléfines (1), pour transformer le produit hydrocarboné obtenu en hydrocarbures à chaîne longue,
avec un premier dispositif de séparation (5) pour séparer le produit hydrocarboné obtenu dans le réacteur pour oléfines (1) en une phase gazeuse et une phase liquide, la phase gazeuse étant reliée par l'intermédiaire d'un conduit avec l'entrée du réacteur d'oligomérisation (8),
avec un deuxième dispositif de séparation (9) pour séparer la phase liquide du produit hydrocarboné en un mélange riche en hydrocarbures en C₆ et en un mélange contenant des hydrocarbures en C₇₊ et des aromates, la zone haute du deuxième dispositif de séparation (9) étant reliée par l'intermédiaire d'un conduit (6) avec l'entrée du réacteur d'oligomérisation (8) et
avec un troisième dispositif de séparation (10) sous la forme d'une colonne de distillation (10) pour séparer le mélange obtenu dans le réacteur d'oligomérisation (8) en un distillat, un flux d'hydrocarbure riche en paraffine et en un flux d'hydrocarbure riche en oléfines,
le pied du troisième dispositif de séparation (10) étant relié par l'intermédiaire d'un conduit (13) avec un réacteur d'hydratation (14) dans lequel du diesel est produit à partir du distillat, en ajoutant de l'hydrogène,
la zone haute du troisième dispositif de séparation (10) étant reliée par l'intermédiaire d'un conduit (11) avec l'entrée du réacteur pour oléfines (1) et
le troisième dispositif de séparation (10) étant relié par l'intermédiaire d'un conduit (12) avec l'entrée du réacteur d'oligomérisation (8).

9. Installation selon la revendication 8, **caractérisé en ce que** le deuxième dispositif de séparation (9) est une colonne de distillation.
